# EUROPEAN PATENT SPECIFICATION

(11) **EP 1 240 261 B1**
(45) Date of publication and mention of the grant of the patent: **18.02.2004**
(21) Application number: 00966316.2
(22) Date of filing: 11.10.2000
(51) Int. Cl.: C09D 165/04, B65D 83/14

(54) **MEDICAMENT DELIVERY DEVICE WITH MOISTURE RESISTANT COATING**
MEDIKAMENTENABGABEVORRICHTUNG MIT EINER FEUCHTIGKEITSBESTÄNDIGEN BESCHICHTUNG
DISPOSITIF DE DIFFUSION DE MEDICAMENT A ENROBAGE RESISTANT A L'HUMIDITE

(30) Priority: 11.10.1999 GB 9923959; 15.07.2000 GB 0017314
(43) Date of publication of application: 18.09.2002
(73) Proprietor: Innovata Biomed Limited, St. Albans AL1 3HW (GB)
(72) Inventor: BRAITHWAITE, Philip, c/o ML Laboratories Plc, Tewkesbury GL20 8NB (GB)
(74) Representative: Harrison Goddard Foote
(86) International application number: PCT/GB2000/003892
(87) International publication number: WO 2001/027210

(56) References cited:
- EP-A- 0 045 522
- EP-A- 0 659 432
- WO-A-00/12163
- WO-A-93/16748
- WO-A-95/15777
- US-A- 4 882 210
- US-A- 4 950 365
- US-A- 5 064 083

## Description

This invention relates to a novel form of reservoir means, such as a medicament capsule and the like and to a delivery device e.g. an inhaler, for use in administering a medicament in such a reservoir means.

Many medicament delivery devices, such as inhalers, make use of medicament in a finely divided powder form. The powder may be located within the delivery device, for instance, in a single storage compartment or in a plurality of single dose locations.

One form of inhaler may make use of medicament powder which is located within a frangible, plastic capsule. In use, the capsule is inserted into the inhaler and operation of the inhaler ruptures from the plastic capsule so that the powder may be extracted from the capsule and inhaled by the user.

A problem encountered with many such devices making use of powdered medicament is that, if moisture comes into contact with the powder, it will tend to make it less free-flowing and therefore render the operation of the device less effective because the correct dose of powder cannot be fully delivered.

Moisture may access the powder via several different mechanisms. These include the passage of the moisture through, for example, the plastic wall of encapsulated powder for those inhalers which make use of capsules loaded with medicament powder. For those inhalers which include a storage compartment loaded with powder and from which a dose of powder is accessed by some form of moving part within the inhaler and then presented to an air passageway for inhalation, moisture can access powder within the storage compartment by finding its way along a gap or gaps between the moving parts. In some inhalers there is the possibility of a "wick" type path being established between the powder in a storage compartment within the inhaler and a location within the inhaler where a dose of medicament is located.

With inhalers where a plurality of single doses of medicament is located within the inhaler, there is again likely to be one or more moving parts, providing gaps along which moisture may travel to access each individual dose of medicament.

It is also possible that moisture can pass through the plastic walls of inhalers and reach the powder contained within the inhaler whether in a single storage compartment or in individual dosage locations.

International Patent Application No WO 00/12163 describes the use of a Parylene coating on the inner surface of the metering chamber which is intended to mitigate the deposition of medicament particles on the inner walls of a metered dose inhaler (MDI) for the delivery of medicament via a pressurised aerosol.

We have now surprisingly found that a moisture resistant coating, e.g. a Parylene coating, may be used as on a medicament reservoir and/or a medicament delivery device, such as an inhaler, to render the device, and especially the medicament chamber, moisture resistant.

According to a first aspect of the invention we provide a reservoir means which means is provided with a moisture resistant coating.

In a preferred embodiment, the reservoir means contains medicament such that the reservoir means may be used in conjunction with a delivery device.

The reservoir means may be any conventionally known reservoir means, such as a bulk medicament reservoir or one or more single dose reservoir means. The reservoir means shall not include a pressurised canister for use in inhalation therapy as described in the prior art. When the reservoir means is a single dose reservoir, such as a capsule, e.g. a conventional gelatin capsule, or a spool and spool carrier as described in International Patent Application No. WO93/16748, the coating may be on the inner walls or the outer walls of the reservoir means. However, preferably, the reservoir means is coated on the outer walls, such that the reservoir means is substantially sealed in the coating and is rendered moisture proof.

According to a further feature of the present invention we provide a medicament delivery device which comprises a medicament reservoir as hereinbefore described.

In an especially preferred embodiment the medicament delivery device is also provided with a moisture resistant coating. Such a coating preferentially covers substantially the whole of the delivery device.

When the medicament reservoir means comprises a bulk reservoir, then the medicament delivery device may preferentially include a metering member. The metering member preferably is also provided with a moisture resistant coating.

According to a yet further feature of the invention we therefore provide a medicament delivery device which comprises a medicament reservoir, a medicament delivery passage and a metering member characterised in that the device is provided with a moisture resistant coating on one or more surfaces. Preferably, the whole of the device is substantially provided with a moisture resistant coating.

The moisture resistant coating may be provided on one or more external or internal surfaces of the body of the medicament delivery device. The moisture resistant coating preferentially coats one or more surfaces of the bulk medicament reservoir. Other surfaces of the body of the medicament delivery device may also be provided with a moisture resistant coating.

The moisture resistant coating may be in the form of any material which is effective to prevent moisture accessing the powder. Typically, it may be applied to those surfaces between which there may be a gap due to relative movement between the surfaces when the inhaler is in use. However, the moisture resistant coating may be applied additionally or alternatively to other surfaces including the whole or part of the external surface of the inhaler in order to prevent moisture passing into the interior of the inhaler through the walls thereof.

The moisture resistant coating should, of course, be sufficiently stable and robust so that damage to the coating during use of the delivery device.

The moisture resistant coating of the invention may be applied to any conventionally known medicament delivery system. However, in a preferred embodiment, the medicament delivery device is an inhaler. Whilst the moisture proof barrier may be applied to any conventionally known inhaler, it is an especially preferred aspect of the invention for the inhaler to be a dry powder inhaler (DPI).

Thus, in a preferred embodiment we provide an inhaler, e.g. a DPI, in which the medicament reservoir is provided with a moisture resistant coating.

Dry powder inhalers are known, such as TECHNOHALER, being developed by Innovata Biomed in the UK. WO 93/16748 describes an inhaler which comprises a disc-like cartridge having a plurality of medicament carrying capsules around its periphery. Each capsule comprises a spool carrier which houses a spool. Each spool has a flange at each end which form a tight slidable fit within the body of the spool carrier. The space left between the body of the spool and the spool carrier is filled with an appropriate medicament.

In a preferred embodiment we provide a dry powder inhaler wherein the medicament reservoir comprises one or more individual medicament capsules, e.g. spool carriers and wherein each medicament capsule is provided with a moisture resistant coating. Preferably, the medicament capsule is sealed in a moisture resistant coating.

A variety of medicaments may be administered by using the inhaler of the invention. Such medicaments are generally (but not limiting), bronchodilators or other antiasthma drugs or antibiotics. Such medicaments include, but are not limited to β₂-agonists, e.g. fenoterol, formoterol, pirbuterol, reproterol, rimiterol, salbutamol, salmeterol and terbutaline; non-selective beta-stimulants such as isoprenaline; xanthine bronchodilators, e.g. theophylline, aminophylline and choline theophyllinate; anticholinergics, e.g. ipratropium bromide; mast cell stabilisers, e.g. sodium cromoglycate and ketotifen; bronchial anti-inflammatory agents, e.g. nedocromil sodium; and steroids, e.g. beclomethasone dipropionate, fluticasone, budesonide and flunisolide; and combinations thereof.

Specific combinations of medicaments which may be mentioned include combinations of steroids, such as, beclomethasone dipropionate, fluticasone, budesonide and flunisolide; and combinations of to β₂-agonists, such as, formoterol and salmeterol. It is also within the scope of this invention to include combinations of one or more of the aforementioned steroids with one or more of the aforementioned β₂-agonists.

Further medicaments may include proteinaceous compounds and/or macromolecules, for example, leuprolide and alpha interferon; hormones, such as insulin, human growth hormone, parathyroid hormone; growth factors, anticoagulants, immunomodulators, cytokines and nucleic acids.

According to a yet further feature of the invention we provide a method of treating a respiratory disorder which comprises the administering of a therapeutically effective amount of a pharmaceutically active agent to a patient suffering from such a disorder.

The moisture resistant coating is preferentially a biocompatible coating. Such coatings include, but are not limited to, sugars.

Polymers of poly-para-xylylenes are known as parylene. This material is a conformal polymer film which has been used in a number of applications, including electronics circuits and sensor, where environmental and dielectric isolation is required.

We further the use of a parylene in the manufacture of a moisture resistant capsule as hereinbefore described.

Parylenes are thermoplastic polymers that are capable of polymerising on surfaces from an active monomer gas, without the presence of a liquid. The process is capable of producing very thin layers of polymer and, indeed, a layer of from 10 to 20 microns may be sufficient to protect inhalers and their parts from ingress of moisture.

The polymerisation process takes place at room temperature without solvents and additives. Since the parylene is applied as a gas it conforms to the topography of the surface which it contacts. Since the process does not involve a liquid phase, there is no pooling and bridging during application. The coating is free of pinholes even if the coating has a thickness of less than one micron. As well as being resistant against moisture, parylene is also resistant against other media including hydrocarbons, acids and blood.

The coating may be applied in a single vacuum-coating operation in a thickness from 0.025 to 75 microns and can be controlled accurately to ± 10% of the final thickness.

## Claims

1. An inhaler which includes a medicament reservoir means which means is provided with a moisture resistant coating **characterised in that** the reservoir means is coated on the outer walls, such that the reservoir means is substantially sealed in the coating and is rendered moisture proof.

2. An inhaler means according to claim 1 **characterised in that** the reservoir means contains a medicament.

3. An inhaler means according to claim 1 **characterised in that** the reservoir means is a bulk medicament reservoir.

4. An inhaler means according to claim 1 **characterised in that** the reservoir means comprises one or more single dose reservoir means.

5. An inhaler according to claim 1 **characterised in that** the reservoir means comprises a spool carrier housing a spool.

6. An inhaler means according to claim 1 **characterised in that** the moisture resistant coating is a biocompatible coating.

7. An inhaler means according to claim 6 **characterised in that** the biocompatible coating is a sugar.

8. An inhaler means according to claim 6 **characterised in that** the moisture resistant coating is a polymer.

9. An inhaler means according to claim 8 **characterised in that** the polymer is a poly-para-xylylene (parylene).

10. An inhaler means according to claim 1 **characterised in that** the moisture resistant coating has a thickness of from 0.025 to 75 microns.

11. An inhaler according to claim 1 **characterised in that** the inhaler is also provided with a moisture resistant coating.

12. An inhaler according to claim 11 **characterised in that** the moisture resistant coating covers substantially the whole of the inhaler.

13. An inhaler according to claim 1 **characterised in that** the inhaler includes a metering member.

14. An inhaler according to claim 1 **characterised in that** the inhaler is also provided with a moisture resistant coating.

15. An inhaler according to claim 1 **characterised in that** the medicament reservoir comprises a single dosage reservoir.

16. An inhaler according to claim 1 **characterised in that** the medicament reservoir comprises a plurality of single dose units housed in a cartridge.

17. An inhaler according to claim 16 **characterised in that** the cartridge is also provided with a moisture resistant coating.

18. An inhaler according to claim 1 **characterised in that** the inhaler is a dry powder inhaler (DPI).

19. The use of an inhaler according to claim 1 in the manufacture of a treatment of a respiratory disorder which comprises the administering of a therapeutically effective amount of a medicament to a patient suffering from such a disorder wherein said medicament is contained in said inhaler.

20. The use of a parylene in the manufacture of an inhaler according to claim 1.

## Patentansprüche

1. Medikamentenabgabevorrichtung mit einer Medikamentenspeichereinrichtung, wobei die Einrichtung mit einem feuchtigkeitsbeständigen Überzug ausgestattet ist, **dadurch gekennzeichnet, daß** die Speichereinrichtung an den äußeren Wänden derart beschichtet ist, daß die Speichereinrichtung im wesentlichen im Überzug abgedichtet ist und feuchtigkeitsdicht gehalten wird.

2. Medikamentenabgabevorrichtung nach Anspruch 1, **dadurch gekennzeichnet, daß** die Speichereinrichtung ein Medikament enthält.

3. Medikamentenabgabevorrichtung nach Anspruch 1, **dadurch gekennzeichnet, daß** die Speichereinrichtung als ein Reservoir für ein unverpacktes Medikament ausgestaltet ist.

4. Medikamentenabgabevorrichtung nach Anspruch 1, **dadurch gekennzeichnet, daß** die Speichereinrichtung eine oder mehrere Einzeldosis-Speichereinrichtungen aufweist.

5. Medikamentenabgabevorrichtung nach Anspruch 1, **dadurch gekennzeichnet, daß** die Speichereinrichtung einen Aufnahmenträger aufweist, welcher eine Aufnahme beherbergt.

6. Medikamentenabgabevorrichtung nach Anspruch 1, **dadurch gekennzeichnet, daß** der feuchtigkeitsbeständige Überzug als ein biokompatibler Überzug ausgestaltet ist.

7. Medikamentenabgabevorrichtung nach Anspruch 6, **dadurch gekennzeichnet, daß** der biokompatible Überzug ein Zucker ist.

8. Medikamentenabgabevorrichtung nach Anspruch 6, **dadurch gekennzeichnet, daß** der feuchtigkeitsbeständige Überzug ein Polymer ist.

9. Medikamentenabgabevorrichtung nach Anspruch 8, **dadurch gekennzeichnet, daß** das Polymer ein Poly-Para-Xylylen (Parylen) ist.

10. Medikamentenabgabevorrichtung nach Anspruch 1, **dadurch gekennzeichnet, daß** der feuchtigkeitsbeständige Überzug eine Dicke von 0,025 bis 75 Mikrometer aufweist.

11. Medikamentenabgabevorrichtung nach Anspruch 1, **dadurch gekennzeichnet, daß** auch die Medikamentenabgabevorrichtung mit einem feuchtigkeitsbeständigen Überzug ausgestaltet ist.

12. Medikamentenabgabevorrichtung nach Anspruch 11, **dadurch gekennzeichnet, daß** der feuchtigkeitsbeständige Überzug im wesentlichen die gesamte Medikamentenabgabevorrichtung bedeckt.

13. Medikamentenabgabevorrichtung nach Anspruch 1, **dadurch gekennzeichnet, daß** sie ein Meßelement enthält.

14. Medikamentenabgabevorrichtung nach Anspruch 1, **dadurch gekennzeichnet, daß** sie ebenfalls mit einem feuchtigkeitsbeständigen Überzug ausgestaltet ist.

15. Medikamentenabgabevorrichtung nach Anspruch 1, **dadurch gekennzeichnet, daß** der Medikamentenspeicher einen Einzeldosis-Speicher aufweist.

16. Medikamentenabgabevorrichtung nach Anspruch 1, **dadurch gekennzeichnet, daß** der Medikamentenspeicher eine Mehrzahl von Einzeldosiseinheiten aufweist, welche in einer Patrone untergebracht sind.

17. Medikamentenabgabevorrichtung nach Anspruch 16, **dadurch gekennzeichnet, daß** die Patrone ebenfalls mit einem feuchtigkeitsbeständigen Überzug ausgestaltet ist.

18. Medikamentenabgabevorrichtung nach Anspruch 1, **dadurch gekennzeichnet, daß** die Medikamentenabgabevorrichtung als eine Trockenpulverabgabevorrichtung (DPI) ausgestaltet ist.

19. Verwendung einer Medikamentenabgabevorrichtung nach Anspruch 1 bei der Herstellung eines Therapeutikums für eine respiratorische Erkrankung, welche die Abgabe einer therapeutisch wirksamen Menge eines Medikaments an einen Patienten umfaßt, welcher unter einer solchen Erkrankung leidet, wobei das Medikament in der Medikamentenabgabevorrichtung enthalten ist.

20. Verwendung eines Parylens bei der Herstellung einer Medikamentenabgabevorrichtung nach Anspruch 1.

## Revendications

1. Inhaleur qui inclut des moyens de réservoir de médicaments, les moyens étant prévus avec un revêtement résistant à l'humidité, **caractérisé en ce que** les moyens de réservoir sont revêtus sur les parois extérieures, de manière à ce que les moyens de réservoir sont sensiblement fermés de manière hermétique dans le revêtement et sont rendus résistants à l'humidité.

2. Moyens d'inhaleur selon la revendication 1, **caractérisés en ce que** les moyens de réservoir contiennent un médicament.

3. Moyens d'inhaleur selon la revendication 1, **caractérisés en ce que** les moyens de réservoir sont des réservoirs de médicaments en vrac.

4. Moyens d'inhaleur selon la revendication 1, **caractérisés en ce que** les moyens de réservoir comprennent un ou plusieurs moyens de réservoirs à dose simple.

5. Inhaleur selon la revendication 1, **caractérisé en ce que** les moyens de réservoir comprennent un porteur de bobine comprenant une bobine.

6. Moyens d'inhaleur selon la revendication 1, **caractérisés en ce que** le revêtement résistant à l'humidité est un revêtement biocompatible.

7. Moyens d'inhaleur selon la revendication 6, **caractérisés en ce que** le revêtement biocompatible est un sucre.

8. Moyens d'inhaleur selon la revendication 6, **caractérisé en ce que** le revêtement résistant à l'humidité est un polymère.

9. Moyens d'inhaleur selon la revendication 8, **caractérisés en ce que** le polymère est un poly-para-xylylène (parylène).

10. Moyens d'inhaleur selon la revendication 1, **caractérisés en ce que** le revêtement résistant à l'humidité a une épaisseur d'environ 0,025 à 75 microns.

11. Inhaleur selon la revendication 1, **caractérisés en ce que** l'inhaleur est également prévu avec un revêtement résistant à l'humidité.

12. Inhaleur selon la revendication 11, **caractérisé en ce que** le revêtement résistant à l'humidité recouvre sensiblement l'intégralité de l'inhaleur.

13. Inhaleur selon la revendication 1, **caractérisé en ce que** l'inhaleur inclut un élément de dosage.

14. Inhaleur selon la revendication 1, **caractérisé en ce que** l'inhaleur est également prévu avec un revêtement résistant à l'humidité.

15. Inhaleur selon la revendication 1, **caractérisé en ce que** le réservoir de médicaments comprend un réservoir à dosage simple.

16. Inhaleur selon la revendication 1, **caractérisé en ce que** le réservoir de médicaments comprend une pluralité d'unités de doses simples logée dans une cartouche.

17. Inhaleur selon la revendication 16, **caractérisé en ce que** la cartouche est également prévue avec un revêtement résistant à l'humidité.

18. inhaleur selon la revendication 1, **caractérisé en ce que** l'inhaleur est un inhaleur à poudre sèche (DPI).

19. Utilisation d'un inhaleur selon la revendication 1 dans la fabrication d'un traitement d'une maladie respiratoire qui comprend l'administration d'une quantité efficace de manière thérapeutique d'un médicament à un patient souffrant d'une telle maladie, **caractérisée en ce que** ledit médicament est contenu dans ledit inhaleur.

20. Utilisation d'un parylène dans la fabrication d'un inhaleur selon la revendication 1.
